Europäisches Patentamt

European Patent Office

Office européen des brevets

(19)

(11) Veröffentlichungsnummer: **0 010 666**
**B1**

(12) **EUROPÄISCHE PATENTSCHRIFT**

(45) Veröffentlichungstag der Patentschrift: **29.04.81**

(21) Anmeldenummer: **79103937.3**

(22) Anmeldetag: **12.10.79**

(51) Int. Cl.³: **C 07 D 201/04,**
**C 07 D 223/10**

(54) **Verfahren zur kontinuierlichen Herstellung von epsilon-Caprolactam durch Beckmann'sche Umlagerung.**

(30) Priorität: **16.10.78 DE 2845019**

(43) Veröffentlichungstag der Anmeldung:
**14.05.80 Patentblatt 80/10**

(45) Bekanntmachung des Hinweises auf die Patenterteilung:
**29.04.81 Patentblatt 81/17**

(84) Benannte Vertragsstaaten:
**BE CH DE FR GB IT NL**

(56) Entgegenhaltungen:
**AT - B - 334 382**
**DE - A1 - 2 547 025**
**DE - A1 - 2 656 182**
**DE - A1 - 2 803 657**
**DL - B - 96 235**
**DL - B - 113 537**
**FR - A - 2 - 141 794**

(73) Patentinhaber: **BASF Aktiengesellschaft**
**Carl-Bosch-Strasse 38**
**D-6700 Ludwigshafen (DE)**

(72) Erfinder: **Horn, Peter, Dr. Dipl.-Chem.**
**Im Bruennel 20**
**D-6945 Hirschberg (DE)**
Erfinder: **Grosskinsky, Otto-Alfred, Dr. Dipl.-Chem.**
**Semmelweisstrasse 8**
**D-6700 Ludwigshafen (DE)**
Erfinder: **Thoma, Richard, Dr.**
**Panoramastrasse 6**
**D-6719 Battenberg (DE)**
Erfinder: **Fuchs, Hugo, Dr. Dipl.-Chem.**
**Egellstrasse 28**
**D-6700 Ludwigshafen (DE)**

Courier Press, Leamington Spa, England.

Verfahren zur kontinuierlichen Herstellung von epsilon-Caprolactam durch Beckmann'sche Umlagerung

Gegenstand der Erfindung ist ein Verfahren zur kontinuierlichen Herstellung von ε-Caprolactam durch Beckmann'sche Umlagerung von Cyclohexanonoxim, das in Lösungsmitteln gelöst ist, die gegen Oleum inert sind und die mit Oleum und Wasser nicht mischbar sind, bei erhöhter Temperatur unter Abführen der Umlagerungswärme durch Verdampfen des Lösungsmittels.

Aus der DE—PS 860 357 ist bereits ein Verfahren zur Umlagerung von Cyclohexanonoxim gelöst in Methylenchlorid bekannt, bei dem man die Cyclohexanonoximlösung im Gegenstrom zu Schwefelsäure führt. Desgleichen ist aus der CH—PS 267 114 ein Verfahren zur Umlagerung von Cyclohexanonoxim bekannt, bei dem eine Cyclohexanonoximlösung und Schwefelsäure gleichzeitig in ein Reaktionsgefäßge leitet und die Reaktionswärme durch Verdampfen des Lösungsmittels abgeletiet wird. Beide Verfahren haben bislang keinen Eingang in die Technik gefunden, da sie mit vielerlei Mängeln behaftet sind. Die Nachteile der beschriebenen Verfahren sind in der englischen Patentschrift 900 801 ausführlich als Stand der Technik abgehandelt worden. So erhält man bei den genannten Verfahren Produkte minderer Qualität, zum anderen besitzen die Verfahren erhebliche technische Mängel, da schnell laufende Rührer bzw. Trennwände eingesetzt werden müssen. Schnell laufende Rührer weisen hohe Energie- und Reparaturkosten auf. Es wurden auch schon Kreislaufverfahren zur Beckmann'schen Umlagerung von Cyclohexanonoxim angewandt (DE—OS 27 39 614). Solche Verfahren haben den Nachteil, daß aufgrund der Abßenkühlung örliche Überhitzungen nicht verhindert werden können. Dies führt zu Ausbeuteminderungen und zudem zur Bildung von unerwünschten Nebenprodukten, die nur schwer zu beseitigen sind.

Es war deshalb die technische Aufgabe gestellt, die Umlagerung von Cyclohexanonoxim in Lösung so zu gestalten, daß sie technisch einfach durchführbar ist und möglichst geringe Mengen an schwer zu beseitigenden Verunreinigungen anfallen.

Diese Aufgabe wird gelöst durch ein Verfahren zur kontinuierlichen Herstellung von ε-Caprolactam durch Beckman'sche Umlagerung von Cyclohexanonoxim, das in Lösungsmitteln gelöst ist, die gegen Oleum inert sind und die mit Oleum und Wasser nicht mischbar sind bei erhöhter Temperatur unter Abführen der Umlagerungswärme durch Verdampfen von Lösungsmitteln, indem man Caprolactam enthaltendes Oleum über eine Förderzone durch eine Mischzone, eine Siedezone und eine Trennzone im Kreis führt, wobei man vor der Förderzone Oleum in dem Maße zugibt, wie es verbraucht wird, in der Mischzone eine Lösung von Cyclohexanonoxim unter hoher Turbulenz zugibt und in der Siedezone durch Verdampfen von Lösungsmittel und Rückführen des Kondensats Umlagerungswärme in an sich bekannter Weise abführt, in der Trennzone das Reaktionsgemisch in eine Lösungsmittelphase, aus der Lösungsmittel in dem Maße wie es anfällt entnommen wird, und Oleum, das Caprolactam enthält, trennt, wobei man das im Oleum gelöste Caprolactam in dem Maße entnimmt, wie es gebildet wird.

Das neue Verfahren hat den Vorteil, daß keine örtlichen Überhitzungen auftreten und die Umlagerungswärme sofort abgeführt wird. Ferner hat das neue Verfahren den Vorteil, daß es einfach durchführbar ist und leicht in den technischen Maßstab übertragbar ist, Darüber hinaus hat das neue Verfahren den Vorteil, daß es mit hervorragenden Ausbeuten verläuft und weniger schwer entfernbare Verunreinigungen gebildet werden.

Erfindungsgemäß führt man Caprolactam enthaltendes Oleum über eine Förderzone durch eine Mischzone über eine Siedezone und eine Trennzone im Kreis. Vor der Förderzone wird Oleum in dem Maße zugegeben, wie es verbraucht wird und in der Mischzone wird in Lösungsmitteln gelöstes Cyclohexanonoxim unter hoher Turbulenz zugeführt. In der anschließenden Siedezone wird durch Verdampfen von Lösungsmittel unter Rückführen des Kondensats die Umlagerungswärme abgeführt. In der darauffolgenden Trennzone wird das Reaktionsgemisch in zwei Phasen, eine Lösungsmittelphase und eine Caprolactam enthaltende Oleumphase getrennt. Aus der Lösungsmittelphase wird das Lösungsmittel in dem Maße, wie es anfällt, entnommen, während aus der Caprolactam enthaltenden Oleumphase Caprolactam gelöst in Oleum in dem Maße wie es gebildet wird, ausgeschleust wird, und die restliche Caprolactam enthaltende Oleumphase wieder der Förderzone nach Ergänzen mit frischem Oleum zugeführt wird.

Vorteilhaft verwendet man als Lösungsmittel Cycloalkane vorzugsweise mit einem Siedepunkt von 50 bis 110°C. Geeignete Cycloalkane sind beispielsweise Cyclopentan, Cyclohexan oder Cycloheptan oder deren Gemische. In der Regel geht man von 10 bis 30 volumenprozentigen Lösungen von Cyclohexanonoxim in den genannten Lösungsmitteln aus. Solche Lösungen werden beispielsweise hergestellt durch Lösen von Cyclohexanonoxim, das von seiner Herstellung noch 3 bis 7 Gew.% Wasser enthält. Vorteilhaft wird vor deren Verwendung aus der Lösung des Cyclohexanonoxim Wasser z.B. durch Dekantieren oder azeotrope Destillation abgetrennt.

Das Caprolactam enthaltende Oleum enthält im allgemeinen 40 bis 60 Gew.% Caprolactam, insbesondere 46 bis 53 Gew.%.

Unter Oleum versteht man freies Schwefeltrioxid enthaltende Schwefelsäure. Es versteht sich, daß die im raschen Kreis umlaufende Flüssigkeit zudem noch Caprolactam gelöst enthält. Vorteilhaft hält man in der aus Oleum und Caprolactam bestehenden Umaufflüssigkeit einen Gehalt an freiem $SO_3$ von 5 bis 15, insbesondere 8 bis 14 Gew.% ein.

Die Zufuhr von Oleum erfolgt vor der Förderzone. Als Förderzone sind für das beschriebene Medium geeignete Pumpen zu verstehen, wie in der Technik übliche Kreiseloder Kolbenpumpen. Anschließend führt man in einer Mischzone die oben genannte Lösung von Cyclohexanonoxim in einem Lösungsmittel unter hoher Turbulenz zu. Die hohe Turbulenz erzielt man vorteilhaft, indem man die Lösung von Cyclohexanonoxim durch Düsenöffnungen axial und radial mit einem Druck von 5 bis 25 bar, insbesondere 10 bis 20 bar in eine sich in Fließrichtung verjüngende Mischzone einbringt und das im Kreis geführte Caprolactam enthaltende Oleum in Höhe der Düsenöffnung seitlich in die Mischzone zuführt. Hierbei wird durch die hervorgerufene Turbulenz eine innige Vermischung der Lösung von Cyclohexanonoxim mit dem Oleum und somit eine rasche Reaktion erzielt.

In einer vorteilhaft unmittelbar anschließenden Siedezone z.B. einen Behälter mit entsprechenden Kühleinrichtungen wird durch die entstehende Umlagerungswärme Lösungsmittel verdampft und die kondensierten Dämpfe wieder zurückgeführt. Hierdurch wird auf einfache Weise die Umlagerungswärme rasch abgefuhrt, so daß an keiner Stelle Überhitzungserscheinungen auftreten. Vorzugsweise hält man Reaktionstemperaturen von 60 bis 110°C ein. Die Temperatur läßt sich durch den Siedepunkt des verwendeten Lösungsmittels leicht regulieren, z.B. durch Anwenden von atmosphärischen, erniedrigtem oder erhötem Druck. Verwendet man das bevorzugte Lösungsmittel Cyclohexan, läßt sich bei atmosphärischem Druck eine Temperatur von 80°C ± 2°C einhalten. Diese Temperatur kann durch Änderung der Druckverhältnisse und damit des Siedepunkts variiert werden.

Es hat sich auch bewährt, wenn das Volumenverhältnis der Gesamtmenge der in einer Zeiteinheit im Kreislauf geführten Mischung aus Oleum und Caprolactam zue Menge an zugefuhrter Cyclohexanonoximlösung 300:1 bis 1:1, vorzugsweise 100:1 bis 3:1 beträgt.

Nach der Siedezone wird das Reaktionsgemisch in eine Trennzone geleitet, wo es sich in eine Lösungsmittelphase und eine Caprolactam enthaltende Oleumphase trennt. Aus der Lösungsmittelphase wird das Lösungsmittel, in dem Maße wie es anfällt, entnommen und vorteilhaft nach einer Wasserwäsche wieder zum Lösen von Cyclohexanonoxim verwendet. Außer der Caprolactam enthaltenden Oleumphase wird Caprolactam enthaltendes Oleum in dem Maße ausgeschleust, wie es anfällt. Die restliche Menge an Caprolactam enthaltendem Oleum wird wieder der Förderzone nach Ergänzen mit Oleum zugeführt. In der Trennzone hält man in der Regel Verweilzeiten von 10 bis 30 Minuten ein, während die mittlere Verweilzeit im gesamten Kreislauf 15 bis 180 Minuten beträgt.

Das so erhaltene Gemisch aus Oleum und Caprolactam wird durch Neutralisation mit Ammoniak in bekannter Weise neutralisiert und aufgearbeitet. Geeignete Verfahren werden beispielsweise beschrieben in Ullmanns Encyklopädie der technischen Chemie, 4. Auflage, Band 9, Seite 100.

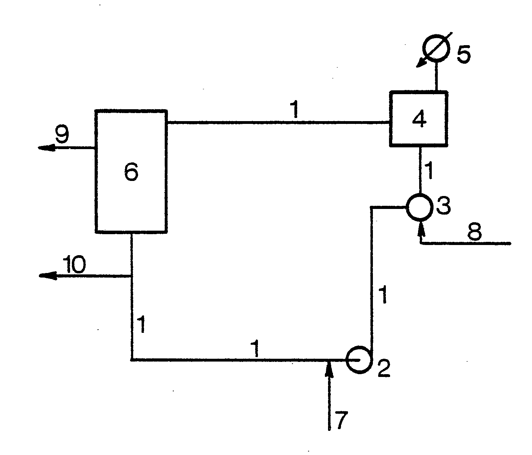

Das Verfahren wird beispielsweise durchgeführt in einem Mischkreis wie in Fig. 1 dargestellt. Der Mischkreise 1 enthält eine Förderzone 2, eine Mischzone 3, eine Siedezone 4 mit einem Kühler 5 sowie eine Trennzone 6. Im Mischkreis wird Oleum bzw. während der Umsetzung Caprolactam enthaltendes Oleum im Kreis gepumpt. Über die Leitung 7 wird frisches Oleum zugeführt und über die Leitung 8 Cyclohexanonoximlösung zudosiert und mit dem umlaufenden Gemisch in der Mischzone 3 innig vermischt. In der nachfolgenden Siedezone 4 wird durch Verdampfen des Lösungsmittels die Umlagerungswärme abgeführt und das im Kühler 5 kondensierende Lösungsmittel zurückgeleitet. In der Trennzone 6 wird das Reaktionsgemisch in eine Lösungsmittelphase getrennt, wobei das Lösungsmittel über Leitung 9 abgeführt wird. Aus der Caprolactam enthaltenden Oleumphase entnimmt man über die Leitung 10 Caprolactam gelöst in Oleum, in dem Maaße, wie es gebildet wird.

Caprolactam, das nach dem Verfahren der Erfindung erhältlich ist, eignet sich zur Herstellung von Polycaprolactam.

Das Verfahren nach der Erfindung sei an folgendem Beispiel erläutert.

Beispiel

Es wird ein Mischkreis entsprechend der Abbildung verwendet, bestehend aus Leitung 1, Kreislaufpumpe 2, Mischdüse 3, Siedebehälter 4 und Kondensator 5 sowie Trennbehälter 6. Das Flüssigkeitsvolumen des Mischkreises beträgt 8 Liter. Mittels der Umlaufpumpe 2 werden stündlich 60 Liter Caprolactam enthaltendes Oleum mit einem Caprolactamgehalt von 46 Gew.% im Kreis gepumpt. Über die Leitung 7 werden stündlich 1,695 Liter Oleum ($SO_3$ Gehalt 28%) und über die Leitung 8 14 Liter einer 20 volumenprozentigen Lösung von Cyclohexanonoxim in Cyclohexan unter einem Druck von 17 bar zugeführt. Bei einem Druck von 1 bar in dem Siedebehälter 4 stellt sich durch das siedende Cyclohexan eine Reaktionstemperatur von 80°C ein. Der Gehalt an freiem Schwefeltrioxid im Umlagerungsgemisch wird auf 11,9 Gew.% eingestellt. Im Trenngefäß 6 wird das Gemisch in zwei Phasen getrennt und über die Leitung 9 das Lösungsmittel abgezogen. Über die Leitung 10 wird Caprolactam enthaltendes Oleum in dem Maße entnommen, wie es gebildet wird. Nach der Aufarbeitung gemäß Ullmanns Encyklopädie der technischen Chemie, 4. Auflage, Band 9, Seite 100 erhält man Caprolactam in einer Ausbeute von 98,5%. Das gewonnene Caprolactam hat in benzolischer Lösung vor der Destillation eine UV-Kennzahl von 109.

## Patentansprüche

1. Verfahren zur kontinuierlichen Herstellung von $\varepsilon$-Caprolactam durch Beckmann'sche Umlagerung von Cyclohexanonoxim das in Lösungsmitteln gelöst ist, die gegen Oleum inert sind und nicht mit Oleum und Wasser mischbar sind, bei erhöhter Temperatur unter Abführen der Umlagerungswärme durch Verdampfen des Lösungsmittels, dadurch gekennzeichnet, daß man Caprolactam enthaltendes Oleum über eine Förderzone durch eine Mischzone über eine Siedezone und eine Trennzone im Kreis führt, wobei man vor der Förderzone Oleum in dem Maße zugibt, wie es verbraucht wird, in der Mischzone in Lösungsmittel gelöstes Cyclohexanonoxim unter hoher Turbulenz zugibt, in der Siedezone durch Verdampfen von Lösungsmittel und Rückführen des Kondensats die Umlagerungswärme in an sich bekannter Weise abführt, in der Trennzone das Reaktionsgemisch in eine Lösungsmittelphase, aus der Lösungsmittel in dem Maß, wie es anfällt, entnommen wird, und eine Caprolactam enthaltende Oleumphase trennt und Caprolactam enthaltendes Oleum in dem Maße entnimmt, wie es gebildet wird.

2. Verfahren nach Anspruch 1, dadurch gekennzeichnet, daß man eine 10 bis 30 volumenprozentige Lösung an Cyclohexanonoxim zuführt.

3. Verfahren nach den Ansprüchen 1 und 2, dadurch gekennzeichnet, daß das Gesamtvolumen des in der Zeiteinheit im Kreis geführten Caprolactam enthaltenden Oleums dem 3- bis 100-fachen Volumen der zugeführten Lösung an Cyclohexanonoxim entspricht.

4. Verfahren nach den Ansprüchen 1 bis 3, dadurch gekennzeichnet, daß das Caprolactam enthaltende Oleum einen Gehalt an freiem $SO_3$ von 5 bis 15 Gew.% hat.

5. Verfahren nach den Ansprüchen 1 bis 4, dadurch gekennzeichnet, daß man Cyclohexan als Lösungsmittel verwendet.

6. Verfahren nach Anspruch 1, dadurch gekennzeichnet, daß man die Cyclohexanonoximlösung durch Düsenöffnungen axial und radial mit einem Druck von 5 bis 25 bar eines sich in Fließrichtung verjüngende Mischzone ein bringt und Caprolactam enthaltendes Oleum in Höhe der Düsenöffnungen seitlich in die Mischzone zuführt.

## Claims

1. A process for the continuous preparation of $\varepsilon$-caprolactam by Beckmann rearrangement of cyclohexanone-oxime, dissolved in a solvent which is inert towards oleum and is immiscible with oleum and water, at an elevated temperature, with removal of the heat of rearrangement by evaporation of the solvent, wherein oleum containing caprolactam is circulated successively through a conveying zone, a mixing zone, a boiling zone and a separating zone, upstream of the conveying zone oleum is added at the rate at which it is consumed, in the mixing zone cyclohexanone-oxime dissolved in solvent is added under turbulent conditions, in the boiling zone the heat of rearrangement is removed in a conventional manner by evaporating the solvent and recycling the condensate, and in the separating zone the reaction mixture is separated into a solvent phase, from which solvent is withdrawn at the rate at which the phase is formed, and a caprolactam-containing oleum phase, which is removed at the rate at which it is formed.

2. A process as claimed in claim 1, wherein a cyclohexanone-oxime solution of from 10 to 30 per cent strength by volume is introduced.

3. A process as claimed in claim 1 or 2, wherein the total volume of the caprolactam-containing oleum recycled per unit time is from 3 to 100 times the volume of cyclohexanone-oxime solution introduced.

4. A process as claimed in any of claims 1 to 3, wherein the caprolactam-containing oleum contains from 5 to 15% by weight of free $SO_3$.

5. A process as claimed in any of claims 1 to 4, wherein cyclohexane is used as the solvent.

6. A process as claimed in claim 1, wherein the cyclohexanone-oxime solution is introduced axially and radially through nozzle orifices, under a pressure of from 5 to 25 bar, into a mixing zone which narrows in the direction of flow, and caprolactam-containing oleum is introduced laterally into the mixing zone, at the level of the nozzle orifices.

## Revendications

1. Procédé de préparation continue d'$\varepsilon$-caprolactame par transposition suivant Beckmann de cyclohexanonoxime dissoute dans des solvants qui sont inertes vis-à-vis de l'oléum et non miscibles avec l'oléum et l'eau, à température élevée, avec évacuation de la chaleur de transposition par vaporisation du solvant, caractérisé par le fait que l'on amène en circuit fermé, par l'intermédiaire d'une zone transporteuse, de l'oléum contenant du caprolactame à travers une zone de mélange, une zone d'ébullition et une zone de séparation, en ajoutant de l'oléum en amont de la zone transporteuse au fur et à mesure qu'il est consommé, en introduisant une solution de cyclohexanonoxime dans un solvant, avec forte turbulence, dans la zone de mélange et en évacuant dans la zone d'ébullition la chaleur de transposition, de manière connue en soi, par vaporisation de solvant et recyclage du condensat, en séparant, dans la zone de séparation, le mélange réactionnel en une phase solvant de laquelle on soutire le solvant au fur et à mesure qu'il est obtenu, et une phase oléum contenant du caprolactame, l'oléum contenant du caprolactame en dissolution étant soutiré au fur et à mesure qu'il est obtenu.

2. Procédé selon la revendication 1, dans lequel on amène une solution à 10 à 30% en volume de cyclohexanonoxime.

3. Procédé selon l'une des revendications 1 et 2, dans lequel le volume total de l'oléum contenant du caprolactame et amené en circuit fermé représente 3 à 100 fois le volume de la solution de cyclohexanonoxime amenée.

4. Procédé selon les revendications 1 à 3, dans lequel l'oléum contenant du caprolactame présente une teneur en $SO_3$ libre comprise entre 5 et 15% en poids.

5. Procédé selon les revendications 1 à 4, dans lequel on utilise du cyclohexane comme solvant.

6. Procédé selon la revendication 1, dans lequel on introduit la solution de cyclohexanonoxime à travers des trous de buse, sous une pression de 5 à 25 bars, axialement et radialement dans une zone de mélange allant se rétrécissant dans le sens d'écoulement, et on amène à ladite zone de mélange, latéralement, au niveau des trous de buse, l'oléum contenant du caprolactame.